# EUROPEAN PATENT APPLICATION

(11) **EP 4 814 108 A1**
(43) Date of publication of application: **30.09.2026**
(21) Application number: 24894037.1
(22) Date of filing: 11.11.2024
(51) Int. Cl.: C12N 1/21, C12N 15/52, C12N 15/60, C12P 7/42

(54) **HYDROGENOPHILUS BACTERIUM TRANSFORMANT CAPABLE OF PRODUCING SALICYLIC ACID**

(30) Priority: 20.11.2023 JP 2023196336
(71) Applicant: Utilization of Carbon Dioxide Institute Co.,Ltd., Tokyo 135-0091 (JP)
(72) Inventor: YUKAWA Hideaki, Tokyo 135-0091 (JP)
(74) Representative: Müller-Boré & Partner Patentanwälte PartG mbB
(86) International application number: PCT/JP2024/040030
(87) International publication number: WO 2025/110045

(57) **Abstract**

A transformant obtained by introducing a salicylate synthase gene described in (a), (b), (c), (d), or (e) below or by introducing an isochorismate synthase gene described in (f), (g), (h), (i), or (j) below and an isochorismate pyruvate lyase gene described in (k), (l), (m), (n), or (o) below into a *Hydrogenophilus* bacterium is capable of efficiently producing salicylic acid utilizing substantially only carbon dioxide as a carbon source.
(a) A DNA comprising the nucleotide sequence of SEQ ID NO: 2
(b) A DNA comprising a nucleotide sequence that has 90% or more identity to SEQ ID NO: 2 and encoding a polypeptide having salicylate synthase activity
(c) A DNA encoding a polypeptide comprising the amino acid sequence of SEQ ID NO: 3
(d) A DNA encoding a polypeptide comprising an amino acid sequence that has 90% or more identity to SEQ ID NO: 3, the polypeptide having salicylate synthase activity
(e) A DNA encoding a polypeptide comprising an amino acid sequence that has 1 to 40 amino acids deleted, substituted, inserted, or added in the amino acid sequence of SEQ ID NO: 3, the polypeptide having salicylate synthase activity
(f) A DNA comprising the nucleotide sequence of SEQ ID NO: 4
(g) A DNA comprising a nucleotide sequence that has 90% or more identity to SEQ ID NO: 4 and encoding a polypeptide having isochorismate synthase activity
(h) A DNA encoding a polypeptide comprising the amino acid sequence of SEQ ID NO: 5
(i) A DNA encoding a polypeptide comprising an amino acid sequence that has 90% or more identity to SEQ ID NO: 5, the polypeptide having isochorismate synthase activity
(j) A DNA encoding a polypeptide comprising an amino acid sequence that has 1 to 40 amino acids deleted, substituted, inserted, or added in the amino acid sequence of SEQ ID NO: 5, the polypeptide having isochorismate synthase activity
(k) A DNA comprising the nucleotide sequence of SEQ ID NO: 6
(l) A DNA comprising a nucleotide sequence that has 90% or more identity to SEQ ID NO: 6 and encoding a polypeptide having isochorismate pyruvate lyase activity
(m) A DNA encoding a polypeptide comprising the amino acid sequence of SEQ ID NO: 7
(n) A DNA encoding a polypeptide comprising an amino acid sequence that has 90% or more identity to SEQ ID NO: 7, the polypeptide having isochorismate pyruvate lyase activity
(o) A DNA encoding a polypeptide comprising an amino acid sequence that has 1 to 10 amino acids deleted, substituted, inserted, or added in the amino acid sequence of SEQ ID NO: 7, the polypeptide having isochorismate pyruvate lyase activity

## Description

### Technical Field

The present invention relates to a *Hydrogenophilus* bacterium transformant having ability to produce salicylic acid, and to a method for producing salicylic acid using the same.

### Background Art

The Paris Agreement, which was adopted in 2015, provides that global emissions of greenhouse gases should be promptly reduced. Under the Agreement, Japan set the goal of reducing her emission of greenhouse gases such as carbon dioxide and methane by 46% compared with year 2013 levels, by the year 2030.

Globally, most chemical production depends on petroleum resources and thus contributes to increased greenhouse gas emissions. Accordingly, reducing dependence on petroleum is a desirable strategy for chemical production, and research and development of biorefineries, which produce green chemicals from biomass, are being actively conducted in various countries. However, biomass saccharification to produce raw materials for microbial fermentation requires complex processes and is also costly. In addition, the use of biomass that can serve as food or feed for chemical production disrupts the stable supply of food and feed. Furthermore, the large-scale use of biomass for chemical production may lead to environmental degradation.

As part of research on decarbonization, gases such as carbon dioxide, methane, and carbon monoxide have attracted attention as more sustainable carbon sources, and techniques for producing valuable substances and biofuels using microorganisms that utilize these gases are the subject of interest. In particular, carbon fixation and efficient utilization of carbon dioxide, a significant contributor to global warming, is highly anticipated.

Salicylic acid is one of the key compounds used in pharmaceuticals, cosmetics, food products, and the like. In pharmaceuticals, salicylic acid is used as a precursor in the manufacture of aspirin and lamivudine (an anti-HIV drug); in cosmetics, salicylic acid is incorporated as an exfoliant into skin care products and facial cleansers. Salicylic acid has recently been reported to be converted into adipic acid, which is the starting material for nylon-6,6, using microbial and chemical methods.

A known industrial method for producing salicylic acid is Kolbe-Schmitt process, in which phenol is reacted with sodium hydroxide to produce sodium phenoxide, which is then reacted with carbon dioxide at high temperature and high pressure to produce sodium salicylate, and sodium salicylate is then converted to salicylic acid under acidic conditions. The starting material phenol is industrially produced using cumene method, which uses petroleum-derived benzene and propylene as raw materials. This method includes complex steps and is a high-temperature, high-pressure chemical industrial process, which consumes large amounts of energy. Therefore, there is a need to shift away from chemical synthesis processes that depend on petroleum resources.

In order to transition from a petroleum-dependent society, which faces concerns over future depletion of petroleum, toward a sustainable society, the production of chemical products by microbial fermentation using renewable, non-edible biomass resources has been attracting attention. However, as mentioned above, the use of biomass involves various challenges, including the need for complex processes, interference with the stable supply of food and feed, and significant environmental burden.

Accordingly, there is a demand for a practical method of producing salicylic acid using microorganisms that does not interfere with the stable supply of food and feed, not imposing environmental burden, and enables production through simpler processes. In particular, there is a need for a practical method that can produce salicylic acid by fixing carbon dioxide.

In microorganisms, the catalytic action of isochorismate synthase converts chorismic acid on the shikimate pathway, which is involved in the synthesis of aromatic amino acids etc., to isochorismic acid, and the catalytic action of isochorismate pyruvate lyase converts isochorismic acid to salicylic acid.

Techniques for producing salicylic acid by fermentation using genetically modified microorganisms have been reported. Patent Literature 1 discloses a method for producing salicylic acid from glucose as a carbon source, which includes introducing an isochorismate synthase gene from *Escherichia coli* or *Pseudomonas aeruginosa* and an isochorismate pyruvate lyase gene from *Pseudomonas aeruginosa* into *Escherichia coli* as well as inhibiting, in the transformant, a pathway that metabolizes phosphoenolpyruvic acid, which is upstream of salicylic acid synthesis, into other compounds.

Non Patent Literature 1 and 2 teach methods for producing salicylic acid from glucose as a carbon source, which includes introducing an isochorismate synthase gene from *Escherichia coli* and an isochorismate pyruvate lyase gene from *Pseudomonas fluorescens* into *Escherichia coli*.

However, these methods are those producing salicylic acid by cultivating microorganisms using sugars, which are costly to produce, and none of the methods are those producing salicylic acid using carbon dioxide as a carbon source.

Patent Literature 2 discloses a method for producing salicylic acid, which includes introducing an isochorismate synthase gene and an isochorismate pyruvate lyase gene from *Pseudomonas aeruginosa* into an anaerobic *Clostridium* bacterium and culturing the transformant. *Clostridium* bacteria use chlorine-containing gaseous substrates as carbon sources for growth, but substantially require carbon monoxide as a carbon source for salicylic acid production. Therefore, the method of Patent Literature 2 is not a method for producing salicylic acid using carbon dioxide as a carbon source, and therefore does not sufficiently contribute to mitigating global warming.

Among bacteria capable of using carbon dioxide as a carbon source for growth, *Cupriavidus necator* H16 strain, *Hydrogenobacter thermophilus* TK-6 strain, and the like have been used in the production of chemical products, but the use of these strains as hosts for salicylic acid production has not been reported.

### Citation List

### Patent Literature

[Patent Literature 1] WO2017/033965 A1
[Patent Literature 2] JP 2018-522536 A

### Non-Patent Literature

[Non Patent Literature 1] Microb Cell Fact., 18(1):18(2019)
[Non Patent Literature 2] Metab Eng., 23:62-69 (2014)

### Summary of Invention

### Technical Problem

The objective of the present invention is to provide a transformant of a *Hydrogenophilus* bacterium that is capable of efficiently producing salicylic acid utilizing carbon dioxide as a sole carbon source, and a method for efficiently producing salicylic acid using this transformant.

### Solution to Problem

The inventor of the present invention has focused on *Hydrogenophilus* bacteria as bacteria capable of fixing carbon dioxide on an industrial scale for the purpose of avoiding the use of high cost raw materials such as sugars and contributing to global warming countermeasures. H*ydrogenophilus* bacteria are bacteria which grow by producing organic substances from carbon dioxide by utilizing hydrogen energy. The growth rate of such bacteria is generally extremely slow, however, the growth rate of *Hydrogenophilus* bacteria is fast, and their ability of fixing carbon dioxide is remarkably higher than that of plants and photosynthetic bacteria.

*Hydrogenophilus* bacteria do not have enzymes for producing salicylic acid. Therefore, it is necessary to introduce the gene of an enzyme which catalyzes a reaction producing salicylic acid to *Hydrogenophilus* bacteria in order to provide capability of producing salicylic acid on an industrial scale to *Hydrogenophilu*s bacteria.

The inventor of the present invention has also found that when a heterologous gene which is expressed within bacteria other than *Hydrogenophilus* bacteria is introduced into *Hydrogenophilus* bacteria, a functioning protein often is not produced or is insufficiently produced. It is generally not practical to divert a gene which can produce a substance within bacteria other than *Hydrogenophilus* bacteria to *Hydrogenophilus* bacteria in order to produce the substance.

Under such circumstances, the present inventor sought to obtain salicylate synthase genes, isochorismate synthase genes, and isochorismate pyruvate lyase genes that are expressible in *Hydrogenophilus* bacteria, and examined genes predicted to encode salicylate synthase, genes predicted to encode isochorismate synthase, and genes predicted to encode isochorismate pyruvate lyase in the genomes of various microorganisms. The present inventor has finally found that the salicylate synthase gene of *Laceyella tengchongensis* enables the expression of functional salicylate synthase in *Hydrogenophilus* bacteria. The present inventor has also found that the isochorismate synthase gene of *Alicyclobacillus contaminans* enables the expression of functional isochorismate synthase in *Hydrogenophilus* bacteria. The present inventor has also found that the isochorismate pyruvate lyase gene of *Pseudomonas aeruginosa* enables the expression of functional isochorismate pyruvate lyase in *Hydrogenophilus* bacteria.

The present inventor has also found that a transformant obtained by introducing the salicylate synthase gene of *Laceyella tengchongensis* into a *Hydrogenophilus* bacterium and a transformant obtained by introducing the isochorismate synthase gene of *Alicyclobacillus contaminans* and the isochorismate pyruvate lyase gene of *Pseudomonas aeruginosa* into a *Hydrogenophilus* bacterium can efficiently produce salicylic acid using carbon dioxide as a sole carbon source.

Salicylate synthase is a bifunctional enzyme having isochorismate synthase activity, which converts chorismic acid to isochorismic acid, and isochorismate pyruvate lyase activity, which converts isochorismic acid to salicylic acid, and catalyzes the reaction to produce salicylic acid from chorismic acid.

The present invention has been completed on the basis of the above findings and provides the following transformant and method for producing salicylic acid.
[1] A transformant obtained by introducing a salicylate synthase gene described in (a), (b), (c), (d), or (e) below into a *Hydrogenophilus* bacterium.
   (a) A DNA comprising the nucleotide sequence of SEQ ID NO: 2
   (b) A DNA comprising a nucleotide sequence that has 90% or more identity to SEQ ID NO: 2 and encoding a polypeptide having salicylate synthase activity
   (c) A DNA encoding a polypeptide comprising the amino acid sequence of SEQ ID NO: 3
   (d) A DNA encoding a polypeptide comprising an amino acid sequence that has 90% or more identity to SEQ ID NO: 3, the polypeptide having salicylate synthase activity
   (e) A DNA encoding a polypeptide comprising an amino acid sequence that has 1 to 40 amino acids deleted, substituted, inserted, or added in the amino acid sequence of SEQ ID NO: 3, the polypeptide having salicylate synthase activity
[2] A transformant obtained by introducing an isochorismate synthase gene described in (f), (g), (h), (i), or (j) below and an isochorismate pyruvate lyase gene described in (k), (l), (m), (n), or (o) below into a *Hydrogenophilus* bacterium.
   (f) A DNA comprising the nucleotide sequence of SEQ ID NO: 4
   (g) A DNA comprising a nucleotide sequence that has 90% or more identity to SEQ ID NO: 4 and encoding a polypeptide having isochorismate synthase activity
   (h) A DNA encoding a polypeptide comprising the amino acid sequence of SEQ ID NO: 5
   (i) A DNA encoding a polypeptide comprising an amino acid sequence that has 90% or more identity to SEQ ID NO: 5, the polypeptide having isochorismate synthase activity
   (j) A DNA encoding a polypeptide comprising an amino acid sequence that has 1 to 40 amino acids deleted, substituted, inserted, or added in the amino acid sequence of SEQ ID NO: 5, the polypeptide having isochorismate synthase activity
   (k) A DNA comprising the nucleotide sequence of SEQ ID NO: 6
   (l) A DNA comprising a nucleotide sequence that has 90% or more identity to SEQ ID NO: 6 and encoding a polypeptide having isochorismate pyruvate lyase activity
   (m) A DNA encoding a polypeptide comprising the amino acid sequence of SEQ ID NO: 7
   (n) A DNA encoding a polypeptide comprising an amino acid sequence that has 90% or more identity to SEQ ID NO: 7, the polypeptide having isochorismate pyruvate lyase activity
   (o) A DNA encoding a polypeptide comprising an amino acid sequence that has 1 to 10 amino acids deleted, substituted, inserted, or added in the amino acid sequence of SEQ ID NO: 7, the polypeptide having isochorismate pyruvate lyase activity
[3] The transformant according to [1] or [2], wherein the *Hydrogenophilus* bacterium is *Hydrogenophilus thermoluteolus*.
[4] A method for producing salicylic acid, the method comprising a step of culturing the transformant according to [1], [2], or [3].

### Advantageous Effects of Invention

Measures to counter the increase in atmospheric carbon dioxide entail reduction of carbon dioxide emissions and fixation of emitted carbon dioxide. In order to reduce carbon dioxide emissions, solar, wind, geothermal, and similar energies are utilized in place of fossil energy. However, the utilization of such energies is not yet extensive enough to repress the buildup of atmospheric carbon dioxide. Consequently, there is need to enhance atmospheric carbon fixation or recycling of emitted carbon dioxide.

Carbon dioxide can be fixed through physical or chemical processes. However, fixation of carbon dioxide utilizing living cells allows the production of organic substances that can be used as food, feed, or fuel. In other words, carbon dioxide itself can be directly converted into valuable substances. Accordingly, the twin problems of global warming due to increased atmospheric carbon dioxide and scarcity of food, feed, and fuel can be solved. Further, in-demand chemical products can be produced while suppressing global warming attributed to increased carbon dioxide emissions.

Salicylic acid is an industrially important aromatic compound. However, the process of chemical synthesis from petroleum feedstock is undesirable from the viewpoint of environmental protection. In contrast, the production of salicylic acid using carbon dioxide-fixing microorganisms can provide a solution to global warming caused by increased carbon dioxide emissions as well as to securing the supply of industrially necessary salicylic acid.

Bacteria that can grow by utilizing the chemical energy generated by the reaction of hydrogen with oxygen and by using carbon dioxide as a sole carbon source, can produce chemical products from a mixture of oxygen, hydrogen, and carbon dioxide gases as raw material. Therefore, the bacteria can achieve the efficient conversion of carbon dioxide into organic compounds and can be cultured in a simple culture medium. Typically, such bacteria grow slowly, but the growth rate of hydrogenoxidizing bacteria, *Hydrogenophilus* bacteria is exceptionally high. The Journal of Mitsubishi Research Institute No. 34 1999 describes *Hydrogenophilus* bacteria as follows: "Their proliferative capacity is so high that their carbon dioxide fixation ability cannot be compared with that of plants, which truly indicates the high carbon dioxide fixation ability of microorganisms".

According to the present invention, introducing a specific salicylate synthase gene into *Hydrogenophilus* bacteria enables the expression of functional salicylate synthase within these bacteria to produce salicylic acid on an industrial scale. Further, introducing a specific isochorismate synthase gene and a specific isochorismate pyruvate lyase gene into *Hydrogenophilus* bacteria enables the expression of functional isochorismate synthase and functional isochorismate pyruvate lyase within these bacteria to produce salicylic acid on an industrial scale.

As described above, *Hydrogenophilus* bacteria have an atypically remarkable carbon dioxide fixation ability among organisms having carbon dioxide fixation ability. Therefore, the present invention has established a pathway for the industrial-scale production of salicylic acid.

### Mode for Carrying Out the Invention

Hereinafter, the present invention will be described in detail.

### (1) Transformants

A first transformant (transformed bacterium) of the present invention is a transformant obtained by introducing the salicylate synthase gene of *Laceyella tengchongensis* or a homologue thereof into a host *Hydrogenophilus* bacterium. That is, the first transformant of the present invention is a *Hydrogenophilus* bacterium transformant having an exogenous gene that is the salicylate synthase gene of *Laceyella tengchongensis* or a homologue thereof.

The salicylate synthase gene used in the present invention does not need to have been previously identified as salicylate synthase gene and may be any DNA encoding a polypeptide having salicylate synthase activity. Salicylate synthase activity refers to an enzymatic activity that converts chorismic acid to salicylic acid.

In the present invention, the salicylate synthase gene may be DNA isolated from naturally occurring bacteria and encoding salicylate synthase, or may be DNA artificially synthesized using methods known to those skilled in the art.

The first transformant of the present invention may have two or more selected from the salicylate synthase gene of *Laceyella tengchongensis* and homologues thereof. This transformant may have not only these genes, but also another exogenous gene or other exogenous genes.

A second transformant (transformed bacterium) of the present invention is a transformant obtained by introducing the isochorismate synthase gene of *Alicyclobacillus contaminans* or a homologue thereof and the isochorismate pyruvate lyase gene of *Pseudomonas aeruginosa* or a homologue thereof into a host *Hydrogenophilus* bacterium. That is, the second transformant of the present invention is a *Hydrogenophilus* bacterium transformant having exogenous genes that are the isochorismate synthase gene of *Alicyclobacillus contaminans* or a homologue thereof and the isochorismate pyruvate lyase gene of *Pseudomonas aeruginosa* or a homologue thereof.

The isochorismate synthase gene used in the present invention does not need to have been previously identified as isochorismate synthase gene and may be any DNA encoding a polypeptide having isochorismate synthase activity. Isochorismate synthase activity refers to an enzymatic activity that converts chorismic acid to isochorismic acid.

The isochorismate pyruvate lyase gene used in the present invention does not need to have been previously identified as isochorismate pyruvate lyase gene and may be any DNA encoding a polypeptide having isochorismate pyruvate lyase activity. Isochorismate pyruvate lyase activity refers to an enzymatic activity that converts isochorismic acid to salicylic acid.

In the present invention, each of the isochorismate synthase gene and the isochorismate pyruvate lyase gene may be DNA isolated from naturally occurring bacteria and encoding the corresponding enzyme, or may be DNA artificially synthesized using methods known to those skilled in the art.

The second transformant of the present invention may have two or more selected from the isochorismate synthase gene of *Alicyclobacillus contaminans* and homologues thereof and two or more selected from the isochorismate pyruvate lyase gene of *Pseudomonas aeruginosa* and homologues thereof. This transformant may have not only these genes, but also another exogenous gene or other exogenous genes.

### Salicylate synthase gene

In the present invention, a DNA comprising the nucleotide sequence of SEQ ID NO: 2 (in particular, a DNA consisting of the nucleotide sequence of SEQ ID NO: 2) can be used as salicylate synthase gene. SEQ ID NO: 2 represents the nucleotide sequence of the salicylate synthase gene of *Laceyella tengchongensis*.

In the present invention, a DNA comprising a nucleotide sequence that has 90% or more, even 95% or more, even 98% or more, even 99% or more identity to SEQ ID NO: 2 (in particular, a DNA consisting of a nucleotide sequence that has 90% or more, even 95% or more, even 98% or more, even 99% or more identity to SEQ ID NO: 2) and encoding a polypeptide having salicylate synthase activity can also be used.

In the present invention, a DNA encoding a polypeptide comprising the amino acid sequence of SEQ ID NO: 3 (in particular, a polypeptide consisting of the amino acid sequence of SEQ ID NO: 3) can also be used as salicylate synthase gene. SEQ ID NO: 3 represents the amino acid sequence of the salicylate synthase of *Laceyella tengchongensis*.

In addition, a DNA encoding a polypeptide comprising an amino acid sequence that has 90% or more, even 95% or more, even 98% or more, even 99% or more identity to SEQ ID NO: 3 (in particular, a polypeptide consisting of an amino acid sequence that has 90% or more, even 95% or more, even 98% or more, even 99% or more identity to SEQ ID NO: 3) and having salicylate synthase activity can be used.

Furthermore, a DNA encoding a polypeptide comprising an amino acid sequence that has 1 to 40, 1 to 20, 1 to 10, 1 to 5, 1 to 4, 1 to 3, or 1 to 2 amino acids, or 1 amino acid deleted, substituted, inserted, or added in the amino acid sequence of SEQ ID NO: 3 (in particular, a polypeptide consisting of an amino acid sequence that has 1 to 40, 1 to 20, 1 to 10, 1 to 5, 1 to 4, 1 to 3, or 1 to 2 amino acids, or 1 amino acid deleted, substituted, inserted, or added in the amino acid sequence of SEQ ID NO: 3) and having salicylate synthase activity can be used.

In the present invention, the presence of salicylate synthase activity in a polypeptide of interest is determined by reacting the polypeptide with chorismic acid and detecting the resulting salicylic acid by liquid chromatography or other techniques.

### Isochorismate synthase gene

In the present invention, a DNA comprising the nucleotide sequence of SEQ ID NO: 4 (in particular, a DNA consisting of the nucleotide sequence of SEQ ID NO: 4) can be used as isochorismate synthase gene. SEQ ID NO: 4 represents the nucleotide sequence of the isochorismate synthase gene of *Alicyclobacillus contaminans*.

In the present invention, a DNA comprising a nucleotide sequence that has 90% or more, even 95% or more, even 98% or more, even 99% or more identity to SEQ ID NO: 4 (in particular, a DNA consisting of a nucleotide sequence that has 90% or more, even 95% or more, even 98% or more, even 99% or more identity to SEQ ID NO: 4) and encoding a polypeptide having isochorismate synthase activity can also be used.

In the present invention, a DNA encoding a polypeptide comprising the amino acid sequence of SEQ ID NO: 5 (in particular, a polypeptide consisting of the amino acid sequence of SEQ ID NO: 5) can also be used as isochorismate synthase gene. SEQ ID NO: 5 represents the amino acid sequence of the isochorismate synthase of *Alicyclobacillus contaminans*.

In addition, a DNA encoding a polypeptide comprising an amino acid sequence that has 90% or more, even 95% or more, even 98% or more, even 99% or more identity to SEQ ID NO: 5 (in particular, a polypeptide consisting of an amino acid sequence that has 90% or more, even 95% or more, even 98% or more, even 99% or more identity to SEQ ID NO: 5) and having isochorismate synthase activity can be used.

Furthermore, a DNA encoding a polypeptide comprising an amino acid sequence that has 1 to 40, 1 to 20, 1 to 10, 1 to 5, 1 to 4, 1 to 3, or 1 to 2 amino acids, or 1 amino acid deleted, substituted, inserted, or added in the amino acid sequence of SEQ ID NO: 5 (in particular, a polypeptide consisting of an amino acid sequence that has 1 to 40, 1 to 20, 1 to 10, 1 to 5, 1 to 4, 1 to 3, or 1 to 2 amino acids, or 1 amino acid deleted, substituted, inserted, or added in the amino acid sequence of SEQ ID NO: 5) and having isochorismate synthase activity can be used.

In the present invention, the presence of isochorismate synthase activity in a polypeptide of interest is determined by reacting the polypeptide with chorismic acid and detecting the resulting isochorismic acid by liquid chromatography or other techniques.

### Isochorismate pyruvate lyase gene

In the present invention, a DNA comprising the nucleotide sequence of SEQ ID NO: 6 (in particular, a DNA consisting of the nucleotide sequence of SEQ ID NO: 6) can be used as isochorismate pyruvate lyase gene. SEQ ID NO: 6 represents the nucleotide sequence of the isochorismate pyruvate lyase gene of *Pseudomonas aeruginosa*.

In the present invention, a DNA comprising a nucleotide sequence that has 90% or more, even 95% or more, even 98% or more, even 99% or more identity to SEQ ID NO: 6 (in particular, a DNA consisting of a nucleotide sequence that has 90% or more, even 95% or more, even 98% or more, even 99% or more identity to SEQ ID NO: 6) and encoding a polypeptide having isochorismate pyruvate lyase activity can also be used.

In the present invention, a DNA encoding a polypeptide comprising the amino acid sequence of SEQ ID NO: 7 (in particular, a polypeptide consisting of the amino acid sequence of SEQ ID NO: 7) can also be used as isochorismate pyruvate lyase gene. SEQ ID NO: 7 represents the amino acid sequence of the isochorismate pyruvate lyase of *Pseudomonas aeruginosa*.

In addition, a DNA encoding a polypeptide comprising an amino acid sequence that has 90% or more, even 95% or more, even 98% or more, even 99% or more identity to SEQ ID NO: 7 (in particular, a polypeptide consisting of an amino acid sequence that has 90% or more, even 95% or more, even 98% or more, even 99% or more identity to SEQ ID NO: 7) and having isochorismate pyruvate lyase activity can be used.

Furthermore, a DNA encoding a polypeptide comprising an amino acid sequence that has 1 to 10, 1 to 5, 1 to 4, 1 to 3, or 1 to 2 amino acids, or 1 amino acid deleted, substituted, inserted, or added in the amino acid sequence of SEQ ID NO: 7 (in particular, a polypeptide consisting of an amino acid sequence that has 1 to 10, 1 to 5, 1 to 4, 1 to 3, or 1 to 2 amino acids, or 1 amino acid deleted, substituted, inserted, or added in the amino acid sequence of SEQ ID NO: 7) and having isochorismate pyruvate lyase activity can be used.

In the present invention, the presence of isochorismate pyruvate lyase activity in a polypeptide of interest is determined by reacting the polypeptide with isochorismic acid and detecting the resulting salicylic acid by liquid chromatography or other techniques.

In the present invention, the identities of nucleotide sequences and amino acid sequences were calculated using GENETYX ver. 17 (GENETYX).

In the present invention, a DNA comprising a nucleotide sequence that has 90% or more but less than 100% identity to the nucleotide sequence of a given DNA and encoding a polypeptide having the same type of activity as the polypeptide encoded by the given DNA is called a "homolog" of the given DNA. A DNA encoding a polypeptide comprising an amino acid sequence that has 90% or more but less than 100% identity to the amino acid sequence of a given polypeptide and having the same type of activity as the given polypeptide is called a homolog of the DNA encoding the given polypeptide. A DNA encoding a polypeptide comprising an amino acid sequence that has 1 to 40 amino acids deleted, substituted, inserted, or added in the amino acid sequence of a given polypeptide and having the same type of activity as the given polypeptide is also called a homolog of the DNA encoding the given polypeptide.

### Hydrogenophilus bacteria

Examples of *Hydrogenophilus* bacteria include *Hydrogenophilus thermoluteolus*, *Hydrogenophilus halorhabdus*, *Hydrogenophilus denitrificans*, *Hydrogenophilus hirschii*, *Hydrogenophilus islandicus*, *Hydrogenophilus thiooxidans, Hydrogenophilus* bacterium strain Mar3 (*Hydrogenophilus* sp. Mar3), and *Hydrogenophilus* bacterium strain Z1038 (*Hydrogenophilus* sp. Z1038). In particular, *Hydrogenophilus thermoluteolus* is preferable because its superior growth rate enables top-level carbon dioxide fixation among carbon dioxide fixing microorganisms.

*Hydrogenophilus* bacteria have been easily isolated from diverse regions everywhere on the earth. A preferable strain of *Hydrogenophilus thermoluteolus* is strain TH-1 (NBRC 14978). *Hydrogenophilus thermoluteolus* strain TH-1 (NBRC 14978) exhibits the highest rapid growth rate among carbon dioxide fixing microorganisms (Agricultural and Biological Chemistry, 41, 685-690 (1977))(It proliferates double per hour.). *Hydrogenophilus thermoluteolus* strain NBRC 14978 is internationally deposited under the Budapest Treaty, and is thus publicly available.

The hosts *Hydrogenophilus* bacteria may be bacteria isolated from nature or genetically modified bacteria derived from bacteria isolated from nature. Genetic modification may be intended, for example, to allow high expression of an introduced salicylate synthase gene, or isochorismate synthase gene and isochorismate pyruvate lyase gene.

Such a genetic modification can be made, for example, by removing (curing) endogenous plasmids in *Hydrogenophilus* bacteria, disruption of genes on the genome of *Hydrogenophilus* bacteria, or other techniques. Methods for removing endogenous plasmids are well known and include, for example, treating with chemicals such as novobiocin, SDS, acriflavine, and ethidium bromide; introducing a plasmid having the same origin of replication as those of endogenous plasmids to destabilize the endogenous plasmids; and utilizing the phenomenon of plasmid incompatibility, such as disrupting factors involved in the plasmid partition system to destabilize endogenous plasmids.

### Method for producing transformant

Next, methods for obtaining transformants by introducing salicylate synthase gene, or isochorismate synthase gene and isochorismate pyruvate lyase gene into *Hydrogenophilus* bacteria are described.

The introduction of these genes into *Hydrogenophilus* bacteria can be carried out using common methods for introducing exogenous genes into bacteria. These genes may be directly introduced into *Hydrogenophilus* bacteria. Alternatively, it may be incorporated into a vector for transformation such as a plasmid vector, viral vector, cosmid, fosmid, or BAC, and then, an obtained vector may be introduced into *Hydrogenophilus* bacteria.

Vectors for transformation should contain a DNA which controls the autonomous replication function within *Hydrogenophilus* bacteria, and examples include broad-host-range vectors pRK415 (GenBank: EF437940.1), pBHR1 (GenBank: Y14439.1), pMMB67EH (ATCC 37622), pCAR1 (NCBI Reference Sequence: NC_004444.1), pC194 (NCBI Reference Sequence:
NC_002013.1), pK18mobsacB (GenBank: FJ437239.1), pUB110 (NCBI Reference Sequence: NC_001384.1), vectors obtained by genetically modifying these vectors (For example, pCAMO-6), and the like.

Among them, pCAMO-6 is preferable. Those skilled in the art can prepare pCAMO-6 by utilizing a gene synthesis service or the like based on its DNA sequence (SEQ ID NO: 1).

Examples of promoters in vectors include tac promoter, lac promoter, trc promoter, or each of promoters OXB1 and OXB11 to OXB20 from Oxford Genetics Ltd. Examples of terminators in vectors include the T1T2 terminator of *Escherichia coli* rRNA operon rrnB, the t0 transcription terminator of bacteriophage λ, T7 terminator, and the like.

Introduction of salicylate synthase gene, or isochorismate synthase gene and isochorismate pyruvate lyase gene into a *Hydrogenophilus* bacterium (transformation) can be carried out by publicly known methods such as calcium chloride method, rubidium chloride method, and electric pulse method (electroporation method).

### (2) Method for producing salicylic acid

The present invention provides a method for producing salicylic acid with use of the transformant of the present invention described above. The method includes a step of culturing the transformant of the present invention, especially, a step of culturing the transformant of the present invention using an inorganic or organic culture medium while supplying a gas containing carbon dioxide, preferably a mixture of gases containing hydrogen, oxygen, and carbon dioxide. The supplied gas is preferably a mixture of gases consisting of hydrogen, oxygen, and carbon dioxide. However, different kinds of gases can be mixed within, to the extent that salicylic acid can be produced efficiently.

*Hydrogenophilus* bacteria can grow using hydrogen as a source of energy and using carbon dioxide as a sole carbon source, and thus, *Hydrogenophilus* bacteria can fix carbon dioxide efficiently when phenol is produced using substantially only carbon dioxide (in particular, by using only carbon dioxide) as a carbon source. Therefore, using an inorganic culture medium that does not contain carbon sources such as organic substances and carbonates is preferable. Namely, carrying out culture using substantially only carbon dioxide (in particular, using only carbon dioxide) as a carbon source is preferable. "Using only carbon dioxide as a carbon source" encompasses cases in which an unavoidable amount of other carbon sources is mixed within.

The pH of the culture medium is preferably 6.2 to 8, more preferably 6.4 to 7.4, and furthermore preferably 6.6 to 7. When the pH is within this range, bacteria grow well and mixed gases dissolves well into the culture medium, and salicylic acid can be produced efficiently.

When batch culture is utilized, mixed gases can be entrapped within an airtight culture container and static culture or shaking culture can be carried out. Shaking culture is preferable in that better dissolution of mixed gases into the culture medium can be achieved. When continuous culture is utilized, mixed gases can be continuously supplied into an airtight culture container and shaking culture can be carried out, or the transformant can be cultured using an airtight culture container while introducing mixed gases into the culture medium by bubbling.

The volume ratio of hydrogen, oxygen, and carbon dioxide (hydrogen: oxygen: carbon dioxide) in the supplied gas mixture is preferably 1.75 to 7.5:1:0.25 to 3, more preferably 5 to 7.5:1:1 to 2, and even more preferably 6.25 to 7.5:1:1.5. When the volume ratio is within this range, bacteria grow well, and salicylic acid can be produced efficiently.

The supply rate of mixed gases or raw material gases can be 10 to 60 L/hour, in particular 10 to 40 L/hour, in particular 10 to 20 L/hour, per 1 L of culture medium. When the supply rate is within this range, transformants grow well and salicylic acid can be produced efficiently, and the amount of wasted mixed gases can be reduced.

The culture temperature is preferably 35 to 55°C, more preferably 37 to 52°C, and even more preferably 50 to 52°C. When the temperature is within this range, transformants grow well, and salicylic acid can be produced efficiently.

Salicylic acid is produced in the medium solution by culturing the transformant in the above-described manner. Salicylic acid can be recovered by collecting obtained medium solution, and it can be further separated from the medium solution by known methods. Such methods include purification techniques using filtration, centrifugation, and various types of chromatography, which may be employed either individually or in combination.

### Examples

The present invention will now be described with reference to examples. The technical scope of the present invention is not limited by the following examples.

### (1) Methods for PCR, electrophoresis, DNA recovery, and seamless cloning in the construction of expression plasmids

PCR, electrophoresis, DNA recovery, and the preparation of plasmid vector pCAMO-6 for seamless cloning in the construction of marker cassettes and expression plasmids were performed using the methods described below.

### (1-1) PCR, electrophoresis, and DNA recovery

PCR was performed in the usual manner using DNA Thermal Cycler manufactured by Life Technologies Inc. and using KOD One PCR Master Mix (manufactured by Toyobo Co., Ltd.) as a reaction reagent. After that, the resulting reaction mixture was subjected to electrophoresis on a 1% agarose gel. Appropriate DNA fragments were recovered from the gel using GEL/PCR Purification Mini Kit (FAVORGEN) as needed.

### (1-2) Preparation of plasmid vector pCAMO-6 for seamless cloning

For seamless cloning, plasmid vector pCAMO-6 was amplified by PCR using the DNA of SEQ ID NO: 1 as a template. The primers shown below were used for PCR.

### Primers for amplification of plasmid vector pCAMO-6

(a-11) 5'-GAATTCGAGCTCCGTCGACA-3' (SEQ ID NO: 8)
(b-11) 5'-ATGCGTTTCTCCTCCAGATC-3' (SEQ ID NO: 9)

The electrophoresis results showed that an approximately 5.2-kbp DNA fragment corresponding to the vector gene was detected. The DNA fragment was recovered from the gel.

### (1-3) Joining of vector pCAMO-6 and insert DNA fragments

The DNA fragment containing vector pCAMO-6 synthesized above was joined to each insert DNA fragment in a one-to-one manner by the recombinase extracted from *Escherichia coli* strain JM109.

*Escherichia coli* strain JM109 was transformed separately with each resulting reaction mixture by heat shock method, applied to LB medium containing 50 µg/mL kanamycin, and cultured at 37°C for 24 hours.

### (1-4) Plasmid extraction and gene sequence confirmation

Strains grown on the LB medium were individually inoculated into 5 mL of liquid LB medium containing 50 µg/mL kanamycin per test tube using a platinum loop, and cultured with shaking at 37°C. Plasmid DNA was extracted from each culture liquid. The analysis of the sequences of the genes inserted in the plasmids was outsourced to Eurofins Genomics K.K. and performed by Sanger method. The results confirmed that the gene sequences were identical to the corresponding sequences in a database.

### (2) Construction of salicylate synthase gene expression plasmids

DNA fragments consisting of the salicylate synthase genes from different bacteria shown below were amplified by PCR.
*Laceyella tengchongensis* (SEQ ID NO: 2)
*Klebsiella pneumoniae* (SEQ ID NO: 10)

The primers shown below were used for PCR.
Primers for amplification of salicylate synthase gene of *Laceyella tengchongensis*
(a-12) 5'-CTGGAGGAGAAACGCATATGAAGAAAACCCCAATGTATGAAG-3' (SEQ ID NO: 11)
(b-12) 5'-GTCGACGGAGCTCGAATTCTCACCCATGTTTCAGCACCAACGTG-3' (SEQ ID NO: 12)

The primers (a-12) and (b-12) contain sequences homologous to regions of vector pCAMO-6.
Primers for amplification of salicylate synthase gene of *Klebsiella pneumoniae*
(a-13) 5'-GATCTGGAGGAGAAACGCATATGAAAATCAGTGAATTTTTACAC-3' (SEQ ID NO: 13)
(b-13) 5'-CGACGGAGCTCGAATTCCTACACCATTAAATAGGGCGCAATGC-3' (SEQ ID NO: 14)

The primers (a-13) and (b-13) contain sequences homologous to regions of vector pCAMO-6.

The electrophoresis results showed that approximately 1.7-kbp DNA fragments corresponding to the salicylate synthase genes from the respective bacterial strains were detected. The DNA fragments were recovered from the agarose gel.

The DNA fragments containing the salicylate synthase genes were individually joined to the DNA fragment containing vector pCAMO-6, and *Escherichia coli* strain JM109 was transformed separately with each resulting plasmid. After that, the plasmids were extracted, and the sequences of the salicylate synthase genes were confirmed.

### (3) Construction of isochorismate synthase gene expression plasmid

A DNA fragment consisting of the isochorismate synthase gene from *Alicyclobacillus contaminans* (SEQ ID NO: 4) was amplified by PCR.

The primers shown below were used for PCR.
(a-14) 5'-GATCTGGAGGAGAAACGCATATGGCTGGACAGCAAGTGATG-3' (SEQ ID NO: 15)
(b-14) 5'-GTCGACGGAGCTCGAATTCTCACGGGCTGTACCTCCCTTCGAGC-3' (SEQ ID NO: 16)

The primers (a-14) and (b-14) contain sequences homologous to regions of vector pCAMO-6.

The electrophoresis results showed that an approximately 1.5-kbp DNA fragment corresponding to the isochorismate synthase gene was detected. The DNA fragment was recovered from the gel.

The DNA fragment containing the isochorismate synthase gene was joined to the DNA fragment containing vector pCAMO-6, and *Escherichia coli* strain JM109 was transformed with the resulting plasmid. After that, the plasmid was extracted, and the sequence of the isochorismate synthase gene was confirmed.

### (4) Construction of isochorismate pyruvate lyase gene expression plasmid

A DNA fragment consisting of the isochorismate pyruvate lyase gene from *Pseudomonas aeruginosa* (SEQ ID NO: 6) was amplified by PCR.

The primers shown below were used for PCR.
(a-15) 5'-GATCTGGAGGAGAAACGCATATGAAAACTCCCGAAGACTGCAC-3' (SEQ ID NO: 17)
(b-15) 5'-GTCGACGGAGCTCGAATTCTCATGCGGCACCCCGTGTCTGGCG-3' (SEQ ID NO: 18)

The primers (a-15) and (b-15) contain sequences homologous to regions of vector pCAMO-6.

The electrophoresis results showed that an approximately 0.3-kbp DNA fragment corresponding to the isochorismate pyruvate lyase gene was detected. The DNA fragment was recovered from the gel.

The DNA fragment containing the isochorismate pyruvate lyase gene was joined to the DNA fragment containing vector pCAMO-6, and *Escherichia coli* strain JM109 was transformed with the resulting plasmid. After that, the plasmid was extracted, and the sequence of the isochorismate pyruvate lyase gene was confirmed.

### (5) Construction of expression plasmid for isochorismate synthase gene and isochorismate pyruvate lyase gene

The isochorismate synthase gene of *Alicyclobacillus contaminans* was amplified using, as a template, the expression vector obtained in the section "(3) Construction of isochorismate synthase gene expression plasmid" and using the following primers.
(a-16) 5'-GGCTCGTATAATGTGTGGAATTGTGAGCGGATAAC-3' (SEQ ID NO: 19)
(b-16) 5'-ATGCGATACTCCTCCTCACGGGCTGTACCTCCCTTCGAGCGGC-3' (SEQ ID NO: 20)

The primer (b-16) contains sequences homologous to the ribosome-binding site and to a region of the isochorismate synthase gene.

The electrophoresis results showed that an approximately 1.5-kbp DNA fragment corresponding to the isochorismate synthase gene was detected. The DNA fragment was recovered from the gel.

The isochorismate pyruvate lyase gene of *Pseudomonas aeruginosa* was amplified using, as a template, the expression vector obtained in the section "(4) Construction of isochorismate pyruvate lyase gene expression plasmid" and using the following primers.
(a-17) 5'-CCGTGAGGAGGAGTATCGCATATGAAAACTCCCGAAGACTGCAC-3' (SEQ ID NO: 21)
(b-17) 5'-CCGGCGGATTTGTCCTACTCAGGAGAGCGTTCACC-3' (SEQ ID NO: 22)

The primer (a-17) contains sequences homologous to the ribosome-binding site and to a region of the isochorismate pyruvate lyase gene.

The electrophoresis results showed that an approximately 0.3-kbp DNA fragment corresponding to the isochorismate pyruvate lyase gene was detected. The DNA fragment was recovered from the gel.

The thus-prepared DNA fragment containing the isochorismate synthase and thus-prepared DNA fragment containing the isochorismate pyruvate lyase were mixed and then fused and amplified using the following primers.
(a-16) 5'-GGCTCGTATAATGTGTGGAATTGTGAGCGGATAAC -3' (SEQ ID NO: 19)
(b-17) 5'-CCGGCGGATTTGTCCTACTCAGGAGAGCGTTCACC-3' (SEQ ID NO: 22)

The electrophoresis results showed that an approximately 1.8-kbp DNA fragment corresponding to a fusion gene of the isochorismate synthase gene and the isochorismate pyruvate lyase gene was detected. The DNA fragment was recovered from the gel.

The DNA fragment containing the fusion gene of the isochorismate synthase gene and the isochorismate pyruvate lyase gene was joined to the DNA fragment containing vector pCAMO-6, and *Escherichia coli* strain JM109 was transformed with the resulting plasmid. After that, the plasmid was extracted, and the sequences of the isochorismate synthase gene and the isochorismate pyruvate lyase gene were confirmed.

### (6) Transformants of Hydrogenophilus thermoluteolus

### (6-1) Gene transfer

*Hydrogenophilus thermoluteolus* strain TH-1 was transformed separately with each plasmid obtained in the sections "(2) Construction of salicylate synthase gene expression plasmids" and "(5) Construction of expression plasmid for isochorismate synthase gene and isochorismate pyruvate lyase gene" by electric pulse method (electroporation), applied to solid LB medium containing 50 µg/mL kanamycin, and cultured at 52°C for 24 hours. Strains grown on the solid LB medium were individually inoculated onto solid LB medium containing 50 µg/mL kanamycin using a platinum loop, and cultured at 52°C for 24 hours. PCR was performed on the grown strains on the solid LB medium to confirm amplification of the fragments inserted in the plasmids. PCR was performed in the usual manner using DNA Thermal Cycler manufactured by Life Technologies Inc. and using KOD One PCR Master Mix (manufactured by Toyobo Co., Ltd.) as a reaction reagent.

The results showed that an approximately 1.7-kbp DNA fragment corresponding to each salicylate synthase gene and an approximately 1.8-kbp DNA fragment corresponding to the combined length of the isochorismate synthase gene and the isochorismate pyruvate lyase gene were separately amplified from each plasmid. The obtained transformants were named as shown in Table 1.

### (6-2) Production of salicylic acid

The *Hydrogenophilus thermoluteolus* transformants prepared as described above were individually inoculated into liquid A medium containing 50 µg/mL kanamycin using a platinum loop, and cultured with shaking at 52°C for 72 hours while a gas mixture of H₂:O₂:CO₂ = 7.5:1:1.5 was supplied. Similarly, *Hydrogenophilus thermoluteolus* strain TH-1 transformed with empty vector pCAMO-6 was cultured. After that, culture supernatants were collected by centrifugation (4°C, 5,000 g, 10 minutes). The concentration of salicylic acid in each culture supernatant was determined by high-performance liquid chromatography (Shimadzu Corporation) under the following conditions.
Mobile phase A: 0.1% aqueous phosphate solution
Mobile phase B: 50% acetonitrile
Flow rate: 1 mL/min
Column: CAPCELL PAK MG II, 5.0 µm, 4.6 mm I.D. × 150 mm
Column temperature: 40°C
PDA temperature: 40°C
PDA: 200 to 300 nm
Reference wavelength: 210 nm

As shown in Table 1, 1.1 mM salicylic acid was detected in the culture supernatant of transformed strain SAS101 carrying the salicylate synthase gene of *Laceyella tengchongensis*. In addition, 0.9 mM salicylic acid was detected in the culture supernatant of transformed strain SAS103 carrying the isochorismate synthase gene of *Alicyclobacillus contaminans* and the isochorismate pyruvate lyase gene of *Pseudomonas aeruginosa*. In contrast, no salicylic acid was detected in the transformed strain SAS102 carrying the salicylate synthase gene of *Klebsiella pneumoniae* or the strain carrying no salicylate synthase gene.

**[Table 1]**

| Strain | Transgene | Concentration of salicylic acid in medium supernatant |
|---|---|---|
| SAS01 | Salicylate synthase gene of *Laceyella tengchongensis* | 1.1 mM |
| SAS02 | Salicylate synthase gene of *Klebsiella pneumoniae* | ND |
| SAS03 | Isochorismate synthase gene of *Alicyclobacillus contaminans* and isochorismate pyruvate lyase gene of *Pseudomonas aeruginosa* | 0.9 mM |
| p-CAMO-6/TH-1 | None | ND |

| | | |
|---|---|---|
| ∺ND: Not Detected | | |

The *Hydrogenophilus* bacterium transformant of the present invention can be prepared by referring to the description of "Examples" of this specification. Other strains mentioned in this specification are publicly available, as they are internationally deposited under the Budapest Treaty, or are possessed by organizations that furnish the strains without any restriction, or are marketed, or can be prepared by those skilled in the art based on this specification.

SEQ ID Nos: 1, 2, 3, 4, 5, 6, 7, and 10 are shown below.
[Chem. 1]
   The first half of SEQ ID NO: 1(pCAMO-6) 
cccatcgactgcacggtgcaccaatgcttctggcgtcaggcagccatcggaagctgtggtatggctgtgcaggt
cgtaaatcactgcataattcgtgtcgctcaaggcgcactcccgttctggataatgttttttgcgccgacatcat
aacggttctggcaaatattctgaaatgagctgttgacaattaatcatcggctcgtataatgtgtggaattgtga
gcggataacaatttcacacaggaaacaggccagttaggcctgctagcagatctggaggagaaacgcatatgctg
atatcggatccgaattcgagctccgtcgacaagcttgcggccgcactgcagcaccatcaccaccatcattgata
agatccggctgctaacaaagcccgaaaggaagctgagttggctgctgccaccgctgagcaataactcgaggcca
atgaggccttcaaataaaacgaaaggctcagtcgaaagactgggcctttcgttttatctgttgtttgtcggtga
acgctctcctgagtaggacaaatccgccgggagcggatttgaacgttgcgaagcaacggcccggagggtggcgg
gcaggacgcccgccataaactgccaggcatcaaattaagcagaaggccatcctgacggatggcctttttgcgtt
tctacaaactccgcggatctaggtgaagatcctttttgataatctcatgaccaaaatcccttaacgtgagtttt
cgttccactgagcgtcagaccccgtagaaaagatcaaaggatcttcttgagatcctttttttctgcgcgtaatc
tgctgcttgcaaacaaaaaaaccaccgctaccagcggtggtttgtttgccggatcaagagctaccaactctttt
tccgaaggtaactggcttcagcagagcgcagataccaaatactgtccttctagtgtagccgtagttaggccacc
acttcaagaactctgtagcaccgcctacatacctcgctctgctaatcctgttaccagtggctgctgccagtggc
gataagtcgtgtcttaccgggttggactcaagacgatagttaccggataaggcgcagcggtcgggctgaacggg
gggttcgtgcacacagcccagcttggagcgaacgacctacaccgaactgagatacctacagcgtgagctatgag
aaagcgccacgcttcccgaagggagaaaggcggacaggtatccggtaagcggcagggtcggaacaggagagcgc
acgagggagcttccagggggaaacgcctggtatctttatagtcctgtcgggtttcgccacctctgacttgagcg
tcgatttttgtgatgctcgtcaggggggcggagcctatggaaaaacgccagcaacgcggcctttttacggttcc
tggccttttgctggccgcggacgtagaaagccagtccgcagaaacggtgctgaccccggatgaatgtcagctac
tgggctatctggacaagggaaaacgcaagcgcaaagagaaagcaggtagcttgcagtgggcttacatggcgata
gctagactgggcggttttatggacagcaagcgaaccggaattgccagctggggcgccctctggtaaggttggga
agccctgcaaagtaaactggatggctttcttgccgccaaggatctgatggcgcaggggatcaagatccgatcaa
gagaggccactaaggcccaggatgaggatcgtttcgcatgattgaacaagatggattgcacgcaggttctccgg
ccgcttgggtggagaggctattcggctatgactgggcacaacagacaatcggctgctctgatgccgccgtgttc
cggctgtcagcgcaggggcgcccggttctttttgtcaagaccgacctgtccggtgccctgaatgaactacagga
cgaggcagcgcggctatcgtggctggccacgacgggcgttccttgcgcagctgtgctcgacgttgtcactgaag
cgggaagggactggctgctattgggcgaagtgccggggcaggatctcctgtcatctcaccttgctcctgccgag
aaagtatccatcatggctgatgcaatgcggcggctgcatacgcttgatccggctacctgcccattcgaccacca
agcgaaacatcgcatcgagcgagcacgtactcggatggaagccggtcttgtcgatcaggatgatctggacgaag
agcatcaggggctcgcgccagccgaactgttcgccaggctcaaggcgcgtatgcccgacggcgaggatctcgtc
gtgactcatggcgatgcctgcttgccgaatatcatggtggaaaatggccgcttttctggattcatcgactgtgg
ccggctgggtgtggcggaccgctatcaggacatagcgttggctacccgtgatattgctgaagagcttggcggcg
aatgggctgaccgcttcctcgtgctttacggtatcgccgctcccgattcgcagcgcatcgccttctatcgcctt
cttgacgagttcttctgaggccatctaggccatgagtaagtctgacggaacggaaagttgtcggttaccagcaa
cgtttcgccctgttggcctcaaaagttcgagtccgttattggtatttccataccaactcaaacgggatataatg
[Chem. 2]
   The latter half of SEQ ID NO: 1(pCAMO-6) 
tgacatgtaacatttctcgattgaggaacgacaatgacagtaccggtcaaagcgcgtgttcaaaagtatcgcga
cgcgctgcgccgctcgggtttgcgtccggttcagatatgggtgccggatacccgccgcccgggctttgcacaag
agtgtcgccgccagtgcctgctcgtgacccaggcagacgccgctgatacgcaactccaacgactcatggatgaa
gccttggcagagattgatggctggcaggcatgatgcgcggcgatttggtaacgatcgttacccagggcgacttt
ggcaaaccaaggccagcgttggtgatccagtccgatctgttcaacgagctcagcagcataaccgtctttcctgt
aaccagtaccattgtggatgccccgttgttgcgcgtcacggttgagccaaccccggaaaacggcttgcacaagc
gatcacaggtgatggtggacaaaattatgaccgtgcggcgggacaaagtcggtcagatcattggacgtatcgat
acgactacgctgacaacgatcgagcgctgcttagctgtcttcttgggaatcgcgaaataaggccatgaaggcct
tacccatcaccgtgcatcacgccgaccgcttcgccactttgcgacaagaaagcaatctgaacgcgctggcgaaa
atggatcggcgctgcccggtctacctgtgaagttcgcgcaaacgtccatcgagacgtacattgccgttggcatc
gatctcgacggcgatttcctctggctaccgatcgcgcgcaccgcttacgcgccctcgacgtagtcgagaaaagc
gtgattgcgtatgttgcgccaactgagcccgtcggtgcgttgtgaccactcgttgtagaccacgcccaaaacca
tcaccccaaacggtgcggaaagtgctactctttcgtcttgccacaacaagaaggagggcgggtggtgatcgaaa
ccggtgctttgagagccaaagaagaccccattgcctgggtccaacagaaagcagccgaggcgttgcaacgccta
gatcaaaagatggcctctcagcccgtgcagatgtacctaccggggttcgatctaggggcctttcccaaccatgt
gaaccgctcctcgctctttgcgcctattgcgcgcggtcgccgcaaatttcatcgacaaacgaagatggtcagcc
gctcggatgctgtgatcgaatacactggagagcagctcgacgaagccgactgcgacctcatgatggcgctcatc
tggtttgcccagaaaaaaccgttaggggagtgggtgacattgaaccgagcgcagctgttgcggcggatcgggcg
cgcgacgggcaagcagaactacgagtggctccatcgcagaatgcgggcgatgaccgaagcgacactctacatcg
aagtgcgcaaatccgacggcagcacacgctacaagatcggctacgccgaggcgtttcacctcatcagcggcttt
gcgttcgatgagaaaagcgacgaatactgctaccaactcgatccccgctgggtacagctcttcagcaaccgcga
atacagcctgatcaacttcgacttgcgattgcagatcaaacggggtcaggacatggcgaaaaccctgcaacgcc
tcgttgctacttcttccgaccccatccagtcttacgaactcgaatggctcaagagcaaaatggaatacaccgga
agaatgcgcgatttcaaagagtcgctctccaaggcgtgcaatgagctgaaacggttgcacatcgtcagtgaata
caagatcactctggacaaaaagaagcaacgggagctgttgaagctctggattcccaaagcgatcacggcataaa
agcggtgcgacactgcacgatcagaaagtattttcagttactcaaacaccgttttttgttgaaaaacaacgaat
tgtcatcggtatagcgtcgcgcacccgagacgaccgacggtgtctggccatcggtatagcgtcgcgctttctcc
acctcttgaacagtcgttcggcatcggtatagcgtcgcgcctgcatcggtatagcgtcgcgctttttcggtata
gcgtcgcgctttttcggtatagcgtcgcgcggttgtcaaaaaatctattattattcaataagttacaagcggtt
tttgaccgtctactatatttctacttatttctactaggaagcgcctgtggataacttcgctcttttctacacag
cccctcagcggcgcaagcgcgccgctgggtagaaaattcctatcgcaacgaactgtcgttcctaaaaataggct
actcggtaaaacaaccgtataaatgatgccgcgtgctcggagtcaactccagaacggcatcggtatagtgtcgc
acttagcaatgtgcgaatatttcatcgttgagccgacttttggagattggtcatgggagatcctcgggttgtcg
aagccttggactcctgttgatagatccagtaatgacctcagaactccatctggatttgttcagaacgctcggtt
gccgccgggcgttttttattggtgagaatccagtctaga
[Chem. 3]
   SEQ ID NO: 2(Salicylate synthase gene of *Laceyella tengchongensis*) 
atgaagaaaaccccaatgtatgaagagacatttgtggcgggcgcgaaagaaccattgaaagtcctgcaccatct
ggcaacaagtgaatttgttaccgattatctattatatgaaggaaatggtgaggttcggattgtcggcaatgtgg
tggccaaaatcgaagtgctacaagacattgtccgctttcaatatgctggtgacaccacagaagagccggcgaag
gacccgctcaaacaagcggagaggtttttgcacacgcttcctttggagcagtggacggcatatggttatgtggg
gtttgatatttgcaaatattactacaactacttcaaatccagtgcggagccaatcttgtgtatgatcgtgccgg
agttcgaacttcgtttcacccaaagcggggtttatataaagtcagtcacccagcaagccgaattgatccaaata
ttggagtcggagattgatttgccaacctatgagcggtatcctctgaaactggactatgctgaccgtgaaaccta
tcaacaaaaagtccaatgtctcattgacaatatccaacaacggcaacttcataagacgatcctctcccgttcag
tcaaggtacatgatcaacttgatcccattctcacttatgcggaggggacaaacgtcaacaacagcgtgcgttcg
tattgccttaaactgggaggcgcaaaagtggtgggcttcagccctgagatcctcatggccgtcgatcgcgagag
atttgtgctcaccaatccacttgccgggacgcgcccgcgcggggacacacctgaacgggatcgacaattgcacg
atgagttattcaccgatgcaaaagaggtgaaagaacacgctttgtctatctggttggcgcaggatgagattcgg
agcgtgtgcgagccagatacggttcgggtgctggattttatgcaagtcaaacgataccgctgcgttcagcattt
gtcttcacgggttggggggacgatcaaagcggaccggacgtcatgggatgcgttgaaagccttatttcccggca
tcaccgtgtcggggattgacaaagcttctgcgttggaatggatttcgcagttggaagaggagccgagaggcatc
tatgctggagcggtaggatggattgattcaagcgatagagcggatttggcgattgcgatacgctctgttttcca
atacggcgatgtcatttcgtttcaagcgggtgccggtatcatgggagaatcgaatcctgagaatgaatacttgg
agacggttaacaaaatgaacaccatgctgtccacgttggtgctgaaacatgggtga
[Chem. 4] 
SEQ ID NO: 3(Salicylate synthase of Laceyella tengchongensis)
MKKTPMYEETFVAGAKEPLKVLHHLATSEFVTDYLLYEGNGEVRIVGNVVAKIEVLQDIV
RFQYAGDTTEEPAKDPLKQAERFLHTLPLEQWTAYGYVGFDICKYYYNYFKSSAEPILCM
IVPEFELRFTQSGVYIKSVTQQAELIQILESEIDLPTYERYPLKLDYADRETYQQKVQCLID
NIQQRQLHKTILSRSVKVHDQLDPILTYAEGTNVNNSVRSYCLKLGGAKVVGFSPEILMAV
DRERFVLTNPLAGTRPRGDTPERDRQLHDELFTDAKEVKEHALSIWLAQDEIRSVCEPDT
VRVLDFMQVKRYRCVQHLSSRVGGTIKADRTSWDALKALFPGITVSGIDKASALEWISQLE
EEPRGIYAGAVGWIDSSDRADLAIAIRSVFQYGDVISFQAGAGIMGESNPENEYLETVNKM
NTMLSTLVLKHG
[Chem. 5]
   SEQ ID NO: 4 (Isochorismate synthase gene of *Alicyclobacillus contaminans*) 
atggctggacagcaagtgatggaacaacagaatccgaccgcgaaaacggtgattgaccggctgcgggaagcggc
gcactgtgcgctgaataccgcccagcatccgcattgcggctggattgtcaccgcgtcgtttcaacgcgcggtgt
cgcgcgagcggctgcggcagctggccggtgcggatccggaagacagtgaacgcatgtactgggcgttgcccggt
gcttccgctgcatatttggcattgggacgggcgctggtgatcacaggcgctggatccggacgggtggaagggct
gcgtcggcagatggacgcgtacgccatccattccgccgatgctgcggacgttcggcaagccgcagccgatgtgc
gctggttcggggggatgtcgtttgctccgaagcggccatcgcaaatgtgggaagggtggcctgaagcgaagttc
gtgctcccccacatcgtagtggaatggcaaggtcaggacaccgcgcgggtggttgcaaccgttgccattcacac
ggcggacgaactggagacgcagttggagggcgttcgccgagagctgcggtggctcgaacggggggcagcggcgt
ccggaatccatgggaggaaagaacccgtgcctccgtgtcgaccgatggcggaggccgcttggtgtgcgcaggtg
tcgagcagtgcggccgagattcgcagcgggcaatacgaaaaagtggtcttagctcggtatgaggcggtgcatgg
catacaccggccgctggccgatgtattggcggagctcgaaggcaagtatcccgacagtcaggtgttcgccgtct
ggcacagtggacagtgctttctcggggcgagccccgagcggctggtctcggtgtccggggagacggtcgatgtg
gactgtttggccggtacggaggcgcgcggccgcactgccgacgaggatgaacacttggcagcacagttgttgga
gagtgccaagaatcgcctggaacacgaggtggttcgggcgtggattgtggaacagttgcaaaccgcagatgtcc
tggatctcacttggccaccgacaccgcaggtgaagcgcctggccaacgtgcagcacttgcatacgcccatccgg
ggacgacggcgggagcagcagtcggtgctcgatttggtggacgagcttcatcccacgcctgccgtggccggtcg
gccacgcgagaaggcgctcgctgtgattgccgagcgcgagtctttggaacgcggttggtacgcgggccccgtgg
ggtggatgaacggccgtggcgaaggtcaattcgcggtcgcgctgcgcagcgctttggtgcggggcgacacggcg
tacgtgttcgcaggcgccggcatcatgggggattcaaatccagcccttgagtgggaggagacgtggatgaaaat
gcgggcggttcgaactgcgctgctgccgctcgaagggaggtacagcccgtga
[Chem. 6]
   SEQ ID NO: 5(Isochorismate synthase of *Alicyclobacillus contaminans*) 
MAGQQVMEQQNPTAKTVIDRLREAAHCALNTAQHPHCGWIVTASFQRAVSRERLRQLAGADPEDSERMYWALPG
ASAAYLALGRALVITGAGSGRVEGLRRQMDAYAIHSADAADVRQAAADVRWFGGMSFAPKRPSQMWEGWPEAKF
VLPHIVVEWQGQDTARVVATVAIHTADELETQLEGVRRELRWLERGAAASGIHGRKEPVPPCRPMAEAAWCAQV
SSSAAEIRSGQYEKVVLARYEAVHGIHRPLADVLAELEGKYPDSQVFAVWHSGQCFLGASPERLVSVSGETVDV
DCLAGTEARGRTADEDEHLAAQLLESAKNRLEHEVVRAWIVEQLQTADVLDLTWPPTPQVKRLANVQHLHTPIR
GRRREQQSVLDLVDELHPTPAVAGRPREKALAVIAERESLERGWYAGPVGWMNGRGEGQFAVALRSALVRGDTA
YVFAGAGIMGDSNPALEWEETWMKMRAVRTALLPLEGRYSP
[Chem. 7]
   SEQ ID NO: 6(Isochorismate pyruvate lyase gene of *Pseudomonas aeruginosa*) 
atgaaaactcccgaagactgcaccggcctggcggacatccgcgaggccatcgaccggatcgacctggatatcgt
ccaggccctcggccgccgcatggactacgtcaaggcggcgtcgcgcttcaaggccagcgaggcggcgattccgg
cgcccgagcgggtcgccgcgatgctccccgagcgcgcccgctgggccgaggagaacggactcgacgcgcccttc
gtcgagggactgttcgcgcagatcatccactggtacatcgccgagcagatcaagtactggcgccagacacgggg
tgccgcatga
[Chem. 8]
   SEQ ID NO: 7 (Isochorismate pyruvate lyase of *Pseudomonas aeruginosa*) 
MKTPEDCTGLADIREAIDRIDLDIVQALGRRMDYVKAASRFKASEAAIPAPERVAAMLPERARWAEENGLDAPF
VEGLFAQIIHWYIAEQIKYWRQTRGAA
[Chem. 9]
   SEQ ID NO: 10(Salicylate synthase gene of *Klebsiella pneumoniae*)

atgaaaatcagtgaatttttacacctggcgttaccagaggaacaatggctgccgacgatttctggcgttttacgccagttcgcag
aagaagagtgttatgtctatgagcgtcaaccctgttggtatttaggcaaagggtgcctggcacggttgcacattaatgccgacgg
aacgcaggcgacatttattgatggtgccggggagcaacaatgggcggtggattccattaccgactgcgcgcgtcgttttatggc
acatcctcaggtcaaaggacgtcgggtttatggacaggttgggttcaactttgcggcgcatgcgcgggggattgcctttaacgc
cggggagtggccgctgctgacgttaaccgttccccgtgaagaacttatttttgaaaagggaaatgtcaccgtttatgcggactcc
gccgacgggggccgacgtttgtgtgagtgggtaaaagaggccagtacaacgacgcagaacgcaccactggcggtggataccg
ccctcaatggcgaggcgtataaacaacaggttgcacgcgccgttgcggagatccgccgtggcgagtatgtcaaagtgattgtct
cgcgcgctattcccctgccatcgcggattgatatgcccgccacgctgttatacgggcggcaggcaaatacacctgtgcgctcgt
ttatgttccgtcaggaaggacgcgaagcgctgggctttagcccggaactggtgatgtcagtgacgggcaataaagtggtcactg
aaccgcttgcgggcacccgcgatcgcatgggaaacccggagcataataaggcgaaagaggcagaactgctgcacgacagtaa
agaggtgcttgagcatatcctttctgtcaaagaagcgattgctgaactggaggccgtttgcctgctgggcagcgtggtggttgaa
gatttaatgtcggttcgccagcgcggcagcgttcagcatctggggtccggcgtgagcggtcagcttgcggaaaacaaggatgc
ctgggatgcgtttaccgtgctgtttccgtcgattaccgcctcaggtatccctaaaaacgctgccctgaacgcgattatgcaaatt
gagaagacgccgcgagagctctattccggcgcaattctgctgctggacgatatgcgcttcgatgcggcgctagttctgcgttcc
gtatttcaggacagccagcgctgctggatacaagcgggggcgggaatcatcgcgcaatctacaccggaacgcgaactgacgg
aaaccagggagaaattagcgagcattgcgccctatttaatggtgtag

### Industrial Applicability

The transformant of the present invention can highly efficiently produce salicylic acid using carbon dioxide as a sole carbon source, thus providing a solution to global warming caused by increased carbon dioxide emissions and contributing to high-efficient industrial production of chemical products.

## Claims

1. A transformant obtained by introducing a salicylate synthase gene described in (a), (b), (c), (d), or (e) below or by introducing an isochorismate synthase gene described in (f), (g), (h), (i), or (j) below and an isochorismate pyruvate lyase gene described in (k), (l), (m), (n), or (o) below into a *Hydrogenophilus* bacterium.
(a) A DNA comprising the nucleotide sequence of SEQ ID NO: 2
(b) A DNA comprising a nucleotide sequence that has 90% or more identity to SEQ ID NO: 2 and encoding a polypeptide having salicylate synthase activity
(c) A DNA encoding a polypeptide comprising the amino acid sequence of SEQ ID NO: 3
(d) A DNA encoding a polypeptide comprising an amino acid sequence that has 90% or more identity to SEQ ID NO: 3, the polypeptide having salicylate synthase activity
(e) A DNA encoding a polypeptide comprising an amino acid sequence that has 1 to 40 amino acids deleted, substituted, inserted, or added in the amino acid sequence of SEQ ID NO: 3, the polypeptide having salicylate synthase activity
(f) A DNA comprising the nucleotide sequence of SEQ ID NO: 4
(g) A DNA comprising a nucleotide sequence that has 90% or more identity to SEQ ID NO: 4 and encoding a polypeptide having isochorismate synthase activity
(h) A DNA encoding a polypeptide comprising the amino acid sequence of SEQ ID NO: 5
(i) A DNA encoding a polypeptide comprising an amino acid sequence that has 90% or more identity to SEQ ID NO: 5, the polypeptide having isochorismate synthase activity
(j) A DNA encoding a polypeptide comprising an amino acid sequence that has 1 to 40 amino acids deleted, substituted, inserted, or added in the amino acid sequence of SEQ ID NO: 5, the polypeptide having isochorismate synthase activity
(k) A DNA comprising the nucleotide sequence of SEQ ID NO: 6
(l) A DNA comprising a nucleotide sequence that has 90% or more identity to SEQ ID NO: 6 and encoding a polypeptide having isochorismate pyruvate lyase activity
(m) A DNA encoding a polypeptide comprising the amino acid sequence of SEQ ID NO: 7
(n) A DNA encoding a polypeptide comprising an amino acid sequence that has 90% or more identity to SEQ ID NO: 7, the polypeptide having isochorismate pyruvate lyase activity
(o) A DNA encoding a polypeptide comprising an amino acid sequence that has 1 to 10 amino acids deleted, substituted, inserted, or added in the amino acid sequence of SEQ ID NO: 7, the polypeptide having isochorismate pyruvate lyase activity

2. The transformant according to claim 1, wherein the *Hydrogenophilus* bacterium is *Hydrogenophilus thermoluteolus*.

3. A method for producing salicylic acid, the method comprising a step of culturing the transformant according to claim 1 or 2.
